# EUROPÄISCHE PATENTSCHRIFT

(11) **EP 0 958 816 B1**
(45) Veröffentlichungstag und Bekanntmachung des Hinweises auf die Patenterteilung: **08.03.2006**
(21) Anmeldenummer: 99102285.6
(22) Anmeldetag: 17.12.1994
(51) Int. Cl.: A61K 31/385, A61P 3/10

(54) **Arzneimittel zur Behandlung des Diabes mellitus sowie seiner Folgeerkrankungen**
Medicament for the treatment of diabetes mellitus as well as its subsequent disorders
Medicament pour le traitement du diabète ainsi que ses maladies ultérieures

(30) Priorität: 21.12.1993 DE 4343593
(43) Veröffentlichungstag der Anmeldung: 24.11.1999
(62) Teilanmeldung aus: 94120045.3
(73) Patentinhaber: VIATRIS GmbH & Co. KG, 61352 Bad Homburg (DE)
(72) Erfinder: Wessel, Klaus, Dr., 61118 Bad Vilbel (DE); Borbe, Harald, Dr., 55127 Mainz (DE); Ulrich, Heinz, Dr., 63843 Niedernberg (DE); Hettche, Helmut, Dr., 63128 Dietzenbach (DE); Bisswanger, Hans, Prof. Dr., 72411 Bodelshausen (DE); Packer, L., Prof. Dr., Orinda, California 94563 (US); Klip, Amira, Prof., Toronto, Ontario M5G 1X8 (CA)
(74) Vertreter: Wibbelmann, Jobst

(56) Entgegenhaltungen:
- C. BONAVENTURA ET AL.: "in vitro a ction of the enantiomers of -lipoic acid" NAUNYN SCHMIEDEBERGS ARCH. PHARMACOL., Bd. 349, 1994, Seite R8 XP002119387
- G. BUCK: "Neurothioct in der therapie der diabetischen Neuropathie." Z. ALLGEM. MED., Bd. 69, Nr. 32, 1993, Seite 945 XP002118219
- J JÖRG ET AL.: "Zur medikamentösen Behandlung der diabetischen Polyneuropathie mit der alpha Liponsäure oder vitamin B-Präparaten." NERVENARTZT, Bd. 59, Nr. 1, 1988, Seiten 36-44, XP002119388

## Beschreibung

Die Erfindung bezieht sich auf Arzneimittel zur Behandlung von Spätkomplikationen und Folgeerkrankungen des Diabetes mellitus vom Typ I und II bzw. von subklinisch vorhandener Insulinresistenz und deren Spätkomplikationen und Folgeerkrankungen.

R-(+)-α-Liponsäure ist das physiologisch vorkommende Enantiomer der 1,2-Dithiocyclopentan-3-valeriansäure.
R-(+)-α-Liponsäure ist ein im Pflanzen- und Tierreich ubiquitäres Coenzym von α-Ketosäuren-Dehydrogenasen (Pyruvatdehydrogenase, α-Ketoglutaratdehydrogenase u.a.m) und wirkt damit an einer Schlüsselstelle des Zucker- und Energiestoffwechsels der Zelle. Es wird in seiner Funktion als intramolekulares Redoxsystem jeweils oxidiert (α-Liponsäure) und reduziert (Dihydroliponsäure).

Als 50/50 Mischung der R-(+)-α-Liponsäure und der S-(-)-α-Liponsäure wird das Racemat eingesetzt zur Behandlung der diabetischen und alkoholischen Polyneuropathie, zur Behandlung von Knollenblätterpilzvergiftungen und von chronischen und alkoholischen Lebererkrankungen.

Es ist bekannt, daß sich die Enantiomeren der α-Liponsäure in verschiedenen pharmakologischen Eigenschaften beispielsweise bezüglich der antiinflammatorischen und analgetischen Wirkung unterscheiden
(Patentschrift EP-A 427 247).
Es ist weiter in der Literatur beschrieben, däß R,S-(+,-)-α-Liponsäure bei Alloxaninduziertem Diabetes im Tiermodell einen blutzuckersenkenden Effekt hat. Dabei ist offen ob dieser Effekt auf eine Beeinflussung der Insulinsekretion oder direkt durch die Aktivierung der Pyruvatdehydrogenase erfolgt. (Natraj C.V. et al.. J. Biosci. Vol. 6(1), 37-46, (1984)).
Stoffwechselabweichungen bei Diabetes wie Hyperglykämie, Ketonämie, Ketonurie reduziertes Glykogen im Gewebe und verminderte Fettsäuresynthese in der Leber werden durch Verabreichung von Liponsäure im Tierexperiment korrigiert.(S.S. Wagh, C.V.Natraj et al J.Biosci.Vol. 11, 59-74(1987))

Ferner ist bekannt, daß dem oxidativen Streß eine promovierende Rolle bei diabetischen Spätkomplikationen zukommt und eine adjuvante Antoxidantientherapie (mit Thiotcacid) zur Regression diabetischer Spätkomplikationen führen kann.(W.Kähler u.a. Innere Medizin 48,(1993) 223-232)

Durch in vitro Experimente mit Thioctsäure (Material der Fa. Calbiochem(Racemat)) ist belegt worden, daß sie die Glukoseaufnahme durch den Muskel steigert. Zeitstudien zeigten, daß im Gegensatz zu dem stimulierenden Effekt von Insulin auf die Glukoseaufnahme die Wirkung von Thioctsäure an Rattendiaphragmen in vitro nur nach verlängerter Inkubation erkennbar ist. Nach Haugaard scheint der Wirkungsmechanismus der Thioctsäure nicht ähnlich dem des Insulins zu sein. Ihr Effekt ist additiv zu der des Insulins. (N. and E.S.Haugaard, Biochim. Biophys. Acta 222,(1970) 583-586). Eine Aussage über eine differenzierte Wirkung von R- oder S-Thioctsäure findet sich jedoch in dieser Literaturstelle ebenfalls nicht.
Der Diabetes mellitus ist eine Erkrankung mit einem Insulindefizit oder einer Resistenz gegenüber der Insulinwirkung (dekompensierte Insulinresistenz). In der Folge treten, auch schon bei noch kompensierter Insulinresistenz (verminderte Insulinwirkung ohne klinisch manifesten Diabetes Typ II) zahlreiche metabolische Störungen insbesondere des Kohlenhydrat- und Fettstoffwechsels auf. Diese Störungen können im Langzeitverlauf zum Koma und zum Tod führen. Sowohl die Insulinresistenz als auch der erhöhte Blutzucker und der gestörte Fettstoffwechsel sind an der Enstehung von Folgeerkrankungen und Spätkomplikationen beteiligt (z. B. Katarakt, Neuropathien, Nephropathien). Der erhöhte Blutzucker kann behandelt werden durch Substitution mit Insulin und - in milden Fällen - durch orale Antidiabetika. Die Insulinresistenz selbst ist bis heute ohne anerkannte therapeutische Interventionsmöglichkeit.
Eine grundlegende Störung beim Diabetes und der Insulinresistenz liegt in der Glukoseaufnahme der Muskelzellen. Hierbei ist es insbesondere im Rahmen der Insulinresistenz von Bedeutung, die Glukoseaufnahme auf andere Weise zu behandeln als durch Insulingabe und durch die Insulinausschüttung stimulierende Pharmaka, sondern durch davon unabhängige Mechanismen (Häring H.U., Mehnert H. Diabetologica 36, 176-182, 1993)

Die nach der zellulären Aufnahme der Glukose notwendige Metabolisierung im Rahmen des mitochondrialen Energiestoffwechsels ist insbesondere bei gestörter Glukoseutilisation im Rahmen der Insulinresistenz, ein weiterer notwendiger Schritt. Ein Schlüsselenzym ist die Pyruvatdehydrogenase.

Diabetiker zeigen eine verstärkte Glykosilierung und Oxidation von Proteinen mit entsprechenden negativen Konsequenzen für den Patienten (Makita Z. et al., Science 258, 651-653, 1992).

Neu ist und für den Fachmann unerwartet und nicht ableitbar war der Befund, dass spezifisch die R-(+)-α-Liponsäure geeignet ist für die Behandlung des Diabetes mellitus bzw. der Insulinresistenz, während die 5-(-)-α-Liponsäure hierzu praktisch nicht verwendbar ist. Eigene Untersuchungen zeigten, dass im Tierversuch überraschenderweise das Schlüsselenzym Pyruvat-Dehydrogenase durch S-(-)-α-Liponsäure gehemmt wurde.

Aufgabe der Erfindung ist daher die Bereitstellung von Arzneimitteln zur Behandlung der kompensierten und der dekompensierten Insulinresistenz und damit assozierter Erkrankungen und Folgeerkrankungen, bzw. von Folgeerkrankungen und Spätkomplikationen des Diabetes mellitus, nämlich Katarakt und Nephropathie. Die Blutzuckeraufnahme ins Gewebe wird gefördert. Dies ist von klinischer Relevanz bei pathologischen Störungen der Regulation der Blutzuckereinstellung wie beim Diabetes mellitus vom Typ I und II bzw. bei Störungen der Insulinsensitivität des Gewebes (Insulinresistenz). Dies gilt in der Monotherapie und in der Kombination mit anderen Arzneimitteln zur Behandlung des Diabetes mellitus bzw. der Insulinresistenz, zum Beispiel orale Antidiabetika und insbesondere Insulin. Ziel der Therapie kann auch eine Einsparung von therapeutisch verabreichtem Insulin bzw. anderen Antidiabetika sein als auch eine Erniedrigung pathologisch erhöhter endogener Insulinspiegel. Weiterhin können auch Spätkomplikationen bzw. Folgeerkrankungen des Diabetes mellitus bzw. der Insulinresistenz durch Behandlung der Grunderkrankungen therapeutisch beeinflusst werden.

Es wurde nun überraschenderweise gefunden, dass sich vorzugsweise die R-(+)-α-Liponsäure als geeignet erweist zur Behandlung von Folgeerkrankungen und Spätkomplikationen des Diabetes mellitus vom Typ I und II, wie Katarakt und Nephropatie bzw. zur Behandlung der subklinisch und klinisch manifesten Insulinresistenz und deren Folgeerkrankungen.

### Pharmakologische Beispiele

### 1.Pyruvatdehydrogenaseaktivität nach chronischer Gabe in verschiedenen Geweben der spontandiabetischen Ratte

### Ergebnisse

Tendenz nach zweifacher Gabe: Erniedrigung durch S-(-)-α-Liponsäure, Erhöhung durch R-(+)-α-Liponsäure

### Versuchsbeschreibung

Spontan-diabetischen Ratten (BB-Wol BB, Firma Moellegard, Dänemark, n=10/Gruppe) wurde nach Manifestation des Diabetes über sieben Tage täglich 0,3 ml neutrale 0,12 M (entsprechend 50 mg/kg Körpergewicht) R-(+)-α-Liponsäure bzw. S-(-)-α-Liponsäure in die Schwanzvene appliziert. Eine Kontrollgruppe erhielt physiologische Kochsalzlösung. Nach sieben Tagen wurden die Tiere getötet. Die Pyruvatdehydrogenaseaktivität wurde aus dem Herzmuskel bestimmt. Das Gewebe wurde homogenisiert.

### Messung der Pyruvat-Dehydrogenaseaktivität

Testprinzip: *Pyruvat + NAD*^{*+*} *+ CoA → Acetyl-CoA* + *CO*_{*2*} *+ NADH + H*^{*+*}

Gemessen wird die Extinktion des reduzierten Coenzyms bei 339 nm in Küvetten mit einem Shimadzu UV 210 Detektor bei 37 °C. Die Isolation des Enzymkomplexes (Köplin R. Ph.D. Thesis, University of Tübingen, FRG, 1988; Stanley C.J., Perham R.N. Biochem. J. 191, 147-154, 1980) und der Enzymassay (Lowry O.H. et al.. J. Biol. Chem. 256, 815-822, 1951) wurden wie beschrieben durchgeführt. Die Proteinmessung erfolgte nach Lowry (Bashan N. et al.. Am. J. Physiol. m262 (Cell Physiol. 31): C682-690, 1992)

### 2.Glukoseaufnahme in Muskelzellen unter Insulin (Klip)

### Ergebnisse

### Glukoseaufnahme

Das R-Enantiomer (2,5 mM) stimuliert die Glukoseaufnahme im Vergleich zum S-Enantiomer um mehr als den Faktor 2, während das S-Enantiomer in gleicher Konzentration eine geringere Wirksamkeit zeigt.

**Glukoseaufnahme in Muskelzellen**

| | | | |
|---|---|---|---|
| **Inkubationszeit (min)** | **Glukoseaufnahme Kontrolle (pmol/mg x min)** | **Glukoseaufnahme R (pmol/mg x min)** | **Glukoseaufnahme S (pmol/mg x min)** |
| 15 | 15,1 ± 0.4 | 16,7 ± 0,6 | 16,3 ± 0,3 |
| 30 | 12,1 ± 0 | 15,9 ± 0,9 | 14,8 ± 0,7 |
| 60 | 16,5 ± 0,4 | 26,1 ± 0,9 | 21.6 ± 0,4 |
| 120 | 15,7 ± 0,6 | 27.0 ± 0,4 | 20,5 ± 0,8 |

**Glukoseaufnahme Muskelzellen in Verbindung mit Insulin (200 nM) R-(+)-α-Liponsäure (2,5 mM)**

| | | | | |
|---|---|---|---|---|
| **Inkubationszeit (min)** | **Glukoseaufna hme Kontrolle (pmollmg x min)** | **Glukoseaufna hme R-(+)-α-Liponsäure (pmol/mg x min)** | **Glukoseaufna hme Insulin (pmollmg x min)** | **Glukoseaufna hme Insulin + R-(+)-α-Liponsäure (pmol/mg x min)** |
| 15 | 20,0 ± 0,9 | 23,2 ± 0,5 | 24,7 ± 0,9 | 25,1 ± 0,6 |
| 30 | 18,1 ± 0,6 | 21,1 ± 0,4 | 21,6 ± 0,4 | 21,1 ± 0,2 |
| 60 | 18,0 ± 0,6 | 25,7 ± 0,5 | 23,7 ± 0,5 | 26,2 ± 0,7 |

Der Effekt des R-Enantiomers ist vergleichbar dem des Insulin (200 nM), jedoch nicht additiv. Im Gegensatz zur R-(+)-α-Liponsäure vermindert das S-Enantiomer die Insulinwirkung.

**Glukoseaufnahme Muskelzellen in Verbindung mit Insulin (200 nM) S-(-)-α-Liponsäure (2,5 mM)**

| | | | | |
|---|---|---|---|---|
| **Inkubationszeit (min)** | **Glukoseaufnahme Kontrolle (pmol/mg x min)** | **Glukoseaufnahme S-(-)-α -Liponsäure (pmol/mg x min)** | **Glukoseaufnahme Insulin (pmol/mg x min)** | **Glukoseaufnahme Insulin + S-(-)-α-Liponsäure (pmol/mg x min)** |
| 15 | 14.5 ± 0,3 | 14,8 ± 0,4 | 17,7 ± 0,3 | 16,0 ± 0,4 |
| 30 | 13,8 ± 0,5 | 13,3 ± 0,4 | 16,3 ± 0.5 | 15,7 ± 0,3 |
| 60 | 15,6 ± 0,5 | 16,0 ± 0,2 | 22,3 ± 0,5 | 19,8 ± 1,1 |

### Versuchsbeschreibung

Die Gewebsmuskelzellen (L6-Myotubes) wurden in 24-Lochplatten angesetzt und differenziert. Nach Inkubation mit den Testsubstanzen erfolgte ein Assay zur Hexose-Aufnahme (³H-2-desoxyglucose, 10 µM, 10 Minuten). Insulin wurde in einer Konzentration von 200 nM zugesetzt, die α-Liponsäure-Enantiomere in einer Konzentration von 2,5 mM. Nach Waschen der Zellen, Zellyse mit NaOH wurde die aufgenommene Radioaktivität im Counter gemessen. Parallele Versuchsansätze mit Cytochalasin-B wurden durchgeführt, um die Glukosetransporter-abhängigen Glukosetranslokation zu bestimmen. Die Ergebnisse können in pmol/min x mg Protein ausgedrückt werden. Die Versuche wurden entsprechend der beschriebenen Methodik durchgeführt (Koivisto U.-M. et al.. J. Biol. Chem. 266, 2615-2621, 1991)

### 4.Einfluß auf die Translokation von Glukosetransportern

### Ergebnisse

R-(+)-α-Liponsäure stimuliert die Translokation von Glukosetransportern (Glut 1 und GLUT 4) vom Zytosol an die Plasmamembran, was einer Aktivierung gleichkommt. S-(-)-α-Liponsäure hat keinen bzw. einen hemmenden Effekt und scheint den Gesamtgehalt an Glukosetransportern in der Zelle zu erniedrigen (GLUT4). Eine Translokation der Glukosetransporter entspricht einer Aktivierung der wichtigsten zellulären Glukoseaufnahmemechanismen. Insulin stimuliert ebenfalls die Glukosetransportertranslokation.

**Einfluß von Enantiomeren der α-Liponsäure (2,5 mM) auf die Translokation von GLUT1 Glukosetransportern in L6-Mytubes**

| | | |
|---|---|---|
| **Behandlung** | **Plasmamembran (relative Einheiten)** | **leichte mikrosomale Fraktion (relative Einheiten)** |
| Kontrolle | 1,00 | 1,00 |
| R-(+)-Lipoat | 1,56 ± 0,25 | 0,46 ± 0,06 |
| S-(-)-Lipoat | 0.93 ± 0,37 | 0,38 ± 0,09 |
| Insulin | 1,07 ± 0,14 | 0,68 ± 0,10 |

**Einfluß von Ennatiomeren der α-Liponsäure (2,5 mM) auf die Translokation von GLUT4 Glukosetransportern in L6-Myotubes**

| | | |
|---|---|---|
| **Behandlung** | **Plasmamembran (relative Einheiten)** | **leichte mikrosomale Fraktion (relative Einheiten)** |
| Kontrolle | 1,00 | 1,00 |
| R-(+)-Lipoat | 1,40 ± 0,08 | 0,59 ± 0,04 |
| S-(-)-Lipoat | 0,84 ± 0,37 | 0,71 ± 0,11 |
| Insulin | 1,38 ± 0,09 | 0,75 ± 0,11 |

### Versuchsbeschreibung

L6-Myotubes werden in 15-cm Schalen (n=4-5) herangezogen und mit 2,5 mM Lipoat in MEM mit 5mM Glukose und 2 % fetales Rinderserum eine Stunde inkubiert. Die Zellen werden abgelöst, homogenisiert und in Fraktionen aufgearbeitet (4° C). Die Aufarbeitung erfolgt in einem HEPES-Puffer mit definiertem Proteaseinhibitorzusatz.. Die Zellfraktionen werden in 6 definierten Zenrifugationsschritten gewonnen. Die Fraktionen werden auf ein 10 % Polyacrylamidgel zur Western Blot Analyse gegeben. Die Glukosetransporter werden mit anti-GLUT1- und anti-GLUT4-Antikörpern bestimmt unter Verwendung von Jod-gelabelten Protein A und autoradiographischer Detektion.

### 5. Einfluß auf den zellulären Gehalt von Glukosetransportern

### Ergebnisse

R-(+)-α-Liponsäure erhöht nach 4 Stunden Inkubation den zellulären Gehalt an GLUT1 und GLUT4 Glukosetransportern. S-(-)-α-Liponsäure hat keinen Einfluß bzw. erniedrigt den zellulären Gehalt.

**Einfluß von Liponsäure-Enantiomeren (2,5 mM) nach 4 Stunden Inkubation auf den Gehalt von Glukosetransportern in L6-Myotubes**

| | | |
|---|---|---|
| **Behandlung** | **GLUT1 (arbiträre Einheiten)** | **GLUT4 (arbiträre Einheiten)** |
| Kontrolle | 1,00 | 1,00 |
| R-(+)-Lipoat | 1,81 ± 0,01 | 1,55 ± 0,24 |
| S-(-)-Lipoat | 1.08 ± 0,01 | 0,79 ± 0,47 |

### Versuchsbeschreibung

L6 Myotubes werden 4 Stunden mit 2,5 mM Liponsäure-Enantiomeren inkubiert in MEM-Medium mit 2 % fetalem Kälberserum und 5 mM Glukose. Der Nachweis der Glukosetransporter erfolgt wie oben beschrieben. Es wird die Membranfraktion nach einmaliger Zentrifugation bei 210.000 g gewonnen.

### 6.Diabetes-bedingte Gewebsschäden

### Ergebnisse

In einem Diabetes-Tiermodell (Streptozotocyn-induzierter Diabetes) wurde nunmehr erstaunlicherweise festgestellt, daß R-Thioctsäure zahlreiche pathologisch veränderte Parameter korrigiert (glykosiliertes Hämoglobin, Proteinoxidation), während das S-Enantiomer eine geringere bis keine Wirkung zeigte. Überraschend war zusätzlich, daß die Mortalität der Tiergruppen in der Gruppe, die dem S-Enantiomer ausgesetzt wurde, erhöht war, während die Mortalität in der Gruppe mit dem R-Enantiomer gegenüber der Kontrolle verringert war.

**Glykosiliertes Hämoglobin**

| | |
|---|---|
| **Versuchsgruppe** | **% glykosiliertes Hämoglobin** |
| Kontrolle | 9,7 ± 1,5 (n=8) |
| R-Thioctsäure Diät | 8,4 ± 1,3 (n=11) |
| S-Thioctsäure Diät | 10,7 ± 2,1 (n=6) |

**Protein-carbonylbildung in Linse und Leber**

| | | |
|---|---|---|
| **Versuchsgruppe** | **nmol carbonyl/mg Protein Linse** | **carbonyl/mg Protein Leber (% der Kontrolle)** |
| Kontrolle | 0,513 ± 0,015 (n=3) | 100 ± 8,9 (n=6) |
| R-Thioctsäure-Diät | 0,429 ± 0,063 (n=3) | 73,2 ± 17,8 (n=6) |
| S-Thioctsäure-Diät | 0,554 ± 0,022 (n=3) | 90,3 ± 10,7 (n=6) |

**Todesrate der Streptozotocin-behandelten Ratten**

| | |
|---|---|
| **Versuchsgruppe** | **% Todesfälle** |
| Kontrolle | 33,3 |
| R-Thioctsäure Diät | 8,3 |
| S-Thioctsäure Diät | 50,0 |

### Versuchsbeschreibung

Weiblichen Wistar Ratten (n=3-6/Gruppe) wurden in getrennten Gruppen 14 Wochen lang Thioctsäure Enatiomere per os mit dem Futter verabreicht (1,65 g/kg Futter).
In der achten Woche wurde bei den Tieren ein Streptozotocin-Diabetes induziert. Sechs Wochen nach Induktion des Diabetes wurden die überlebenden Tiere getötet. Gewebe wurden entnommen und analysiert.

R-(+)-α-Liponsäure kann somit als ein hochspezifischer, wirksamer Arzneistoff zur Behandlung von Katarakt und Nephropathie als Folgeerkrankungen und Spätkomplikationen des Diabetes mellitus vom Typ I und II bzw. bei Störungen der Insulinsensitivität des Gewebes (Insulinresistenz) gelten.

Ebenso können R-(+)Dihydroliponsäure, die Metaboliten z.B. Bisnor- und Tetranorliponsäure sowie deren Salze, Ester und Amide eingesetzt werden.

Als Indikation für die Anwendung von Arzneimitteln, die die genannten Stoffe enthalten, kommen somit in Frage:
- subklinische und klinisch manifeste Insulinresistenz und deren Folgeerkranbkungen (kompensierte und dekompensierte Insulinresistenz)
- Katarakt
- Nephropathien

Die Herstellung der R-(+)-α-Liponsäure, R-(-)-Dihydroliponsäure oder deren Metabolite(z.B. Bisnor - oder teranorliponsäure) sowie deren Salze, Ester, Amide erfolgt in bekannter Weise (siehe beispielsweise auch DE-OS 41 37 773).

Die Erfindung bezieht sich auf die Anwendung von Arzneimitteln zur Behandlung oben genannter Erkrankungen, die, die optisch reine R-(+)-α-Liponsäure, R-(-)-Dihydroliponsäure oder der Metabolite sowie deren Salze, Ester, Amide enthalten.

### Galenische Beispiele

Die in der Patentschrift angegebenen Gewichtsmengen beziehen sich jeweils auf das reine optische Isomer, nicht auf die Salze. Bei Verwendung von Salzen, Estern oder Amiden sind die Gewichtsangaben entsprechend den geänderten Molgewichten anzupassen.

Die Herstellung der Salze erfolgt in bekannter Weise (siehe auch Patentschrift EP-A 901213405). Die pharmazeutischen Zubereitungen enthalten im allgemeinen 5 mg bis 3 g der erfindungsgemäß angewendeten Verbindungen als Einzeldosis. Die erreichten Wirkspiegel im Körper sollen nach Mehrfachgabe zwischen 0,1 und 100 mg/kg Körpergewicht liegen.
Die Verabreichung erfolgt in Form von Tabletten, Kautabletten, Lutschtabletten, Pillen, Kapseln, Granulaten, Dragees, Brausetabletten, Brausepulver, Fertigtrinklösungen, flüssigen Formen zur parenteralen Applikation sowie Aerosolen. Fertigtrinklösungen und flüssige Formen zur parenteralen Applikation können alkholische und wässrige Lösungen, Suspensionen und Emulsionen sein.

Bevorzugte Anwendungsformen sind zum Beispiel Tabletten, die zwischen 10 mg und 2 g sowie Lösungen, die zwischen 1 mg und 200 mg/ml Flüssigkeit aktive Substanz enthalten.

Als Einzeldosen des Wirkstoffes sind beispielsweise zu nennen:
a. orale Formen: 10 mg - 3 g
b. parenterale Formen (intravenös oder intramuskulär): 10 mg - 12 g
c. Inhalationsmittel: 10 mg - 2 g

Die Dosen a) bis c) können beispielsweise 1- bis 6 mal täglich oder als Dauerinfusion gegeben werden.

### Ausführungsbeispiele:

### Beispiel 1:

### Tabletten mit 100 mg R-(+)-α-Liponsäure

250 g R-(+)-α-Liponsäure werden mit 750 g mikrokristalliner Cellulose gleichmäßig verrieben. Nach dem Sieben der Mischung werden 250 g Stärke (Starch 1500/Colorcon), 732,5 g Lactose, 15 g Magnesiumstearat und 2,5 g hochdisperses Siliciumdioxid zugemischt und die Mischung zu Tabletten mit Gewicht 800,0 mg verpreßt.
Eine Tablette enthält 100 mg R-(+)-α-Liponsäure. Gegebenenfalls können die Tabletten nach üblichen Verfahren mit einem magensaftlöslichen oder magensaftpermeablen Filmüberzug versehen werden.

### Beispiel 2:

### Ampullen mit 250 mg R-(+)-α-Liponsäure als Trometamolsalz in 10 ml

250 g R-(+)-α-Liponsäure werden zusammen mit 352,3 g Trometamol (2-Amino-2-(hydroxymethyl)-1,3-propandiol) in einer Mischung aus 9 Liter Wasser für Injektionszwecke und 200 g 1,2-Propylenglykol unter Rühren gelöst. Die Lösung wird mit Wasser für Injektionszwecke auf 10 Liter aufgefüllt und anschließend durch ein Membranfilter der Porenweite 0,2 µm mit Glas Faservorfilter filtriert. Das Filtrat wird unter aseptischen Bedingungen zu 10 ml in sterilisierte 10 ml Ampullen abgefüllt.
Eine Ampulle enthält in 10 ml lnjektionslösung 250 mg R-(+)-α-Liponsäure als Trometamolsalz.

### Beispiel 3:

### Ampullen mit 250 mg R-(-)-Dihydroliponsäure in 10 ml Injektionslösung

60 mg Trometamol und 1 g Ethylendiamintetraessigsäure, Dinatriumsalz werden in 1,8 Liter Wasser für Injektionszwecke gelöst. Die Lösung wird 30 Minuten lang mit Stickstoff begast. Unter weiterer Begasung mit Stickstoff werden in der Mischung 2 g Natriumdisulfit und anschließend 50 g R-(-)-Dihydroliponsäure gelöst. Die Lösung wird mit Wasser für Injektionszwecke, das mit Stickstoff gegast wurde, auf ein Volumen von 2 Liter aufgefüllt. Nach sorgfältigem Mischen wird die Lösung über einen Membranfilter der Porenweite 0,2 µm filtriert und das Filtrat unter aseptischen Bedingungen und unter Vor- und Nachbegasung mit Stickstoff in Ampullen zu 10 ml Füllvolumen abgefüllt.
Eine Ampulle enthält in 10 ml Lösung 250 mg R-(-)-Dihydroliponsäure als Trometamolsalz.

### Literatur

1. Natraj C.V. et al.. J. Biosci. Vol. 6(1), 37-46, 1984
2. Häring H.U., Mehnert H. Diabetologica 36, 176-182, 1993
3. Makita Z. et al.. Science 258, 651-653, 1992
4. Köplin R. Ph.D. Thesis, University of Tübingen, FRG, 1988
5. Stanley C.J., Perham R.N. Biochem. J. 191, 147-154, 1980
6. Lowry O.H. et al.. J. Biol. Chem. 256, 815-822, 1951
7. Bashan N. et al.. Am. J. Physiol. m262 (Cell Physiol. 31): C682-690, 1992
8. Koivisto U.-M. et al.. J. Biol. Chem. 266, 2615-2621, 1991

## Patentansprüche

1. Verwendung von R-(+)-α-Liponsäure, R-(-)-Dihydroliponsäure oder der Metabolite sowie deren Salze, Ester, Amide zur Herstellung von Arzneimitteln zur Behandlung von Katarakt oder Nephropathie als Folgeerkrankung oder Spätkomplikation von Diabetes mellitus und Insulinresistenz.

2. Verwendung nach Anspruch 1, umfassend eine Kombination von R-(+)-α-Liponsäure, R-(-)-Dihydroliponsäure oder der Metabolite sowie deren Salze, Ester, Amide mit mindestens einem anderen Antidiabetikum.

3. Verwendung nach Anspruch 2, wobei das andere Antidiabetikum Insulin ist.

## Claims

1. Use of R-(+)-α-lipoic acid, R-(-)dihydrolipoic acid or the metabolites as well as their salts, esters, amides for the preparation of a medicament for the treatment of cataract or nephropathy as sequelae or late complications of diabetes mellitus and insulin resistence.

2. Use according to claim 1, comprising a combination of R-(+)-α-lipoic acid, R-(-)dihydrolipoic acid or the metabolites as well as their salts, esters, amides with at least one other antidiabetic.

3. Use according to claim 2, wherein the other antidiabetic is insulin.

## Revendications

1. Utilisation d'acide R-(+)-alpha-lipoïque, d'acide R-(-)-dihydrolipoïque ou de leurs métabolites et de leurs sels, esters, amides, pour la préparation de médicaments destinés au traitement de la cataracte ou des néphropathies en tant que maladies ou complications tardives liées au diabète sucré et à l'insulinorésistance.

2. Utilisation selon la revendication 1, comprenant un composé contenant de l'acide R-(+)-alpha-lipoïque, de l'acide R-(-)-dihydrolipoïque ou leurs métabolites et leurs sels, esters, amides, et au moins un autre antidiabétique.

3. Utilisation selon la revendication 2, l'autre antidiabétique étant de l'insuline.
